# EUROPÄISCHE PATENTSCHRIFT

(11) **EP 2 632 523 B1**
(45) Veröffentlichungstag und Bekanntmachung des Hinweises auf die Patenterteilung: **12.07.2017**
(21) Anmeldenummer: 11805378.4
(22) Anmeldetag: 14.10.2011
(51) Int. Cl.: A61M 16/04, A61F 2/20

(54) **VORRICHTUNG IN FORM EINER TRACHEALKANÜLE ODER EINER PROTHESE ZUR WIEDERHERSTELLUNG DER STIMME ZUM EINSETZEN IN EIN TRACHEOSTOMA**
DEVICE IN THE FORM OF A TRACHEAL CANNULA OR A PROSTHESIS FOR RESTORING THE VOICE, TO BE INSERTED IN A TRACHEOSTOMA
DISPOSITIF SOUS FORME DE CANULE TRACHÉALE OU D'UNE PROTHÈSE POUR RECONSTITUER LA VOIX À EMPLOYER DANS UNE TRACHÉOSTOMIE

(30) Priorität: 29.10.2010 DE 102010049896
(43) Veröffentlichungstag der Anmeldung: 04.09.2013
(73) Patentinhaber: Primed Halberstadt Medizintechnik GmbH, 38820 Halberstadt (DE)
(72) Erfinder: LEIBITZKI, Harry, 38889 Blankenburg (DE); SÜSS, Steffen, 38820 Halberstadt (DE)
(74) Vertreter: Donath, Dirk
(86) Internationale Anmeldenummer: PCT/DE2011/001868
(87) Internationale Veröffentlichungsnummer: WO 2012/055390

(56) Entgegenhaltungen:
- EP-A1- 0 551 198
- WO-A1-87/06817
- DE-A1-102005 021 470
- DE-U1-202010 002 803

## Beschreibung

Die vorliegende Erfindung betrifft eine Vorrichtung in Form einer Trachealkanüle oder einer Prothese zur Wiederherstellung der Stimme zum Einsetzen in ein Tracheostoma.

Tracheostoma- Prothesen (auch Trachealkanülen oder Tracheostoma-Tuben genannt) zur Behandlung kehlkopfloser (laryngektomierter) Patienten mit eröffneter Kehle (s.g. Tracheostoma) sind seit Jahrzehnten bekannt.
Neben der Verwendung von reinen Trachealkanülen, bspw. zur Beatmung eines Patienten über eine Öffnung unterhalb des Kehlkopfes, kann gemäß dem Stand der Technik zur Erzeugung einer neuen Stimme bei Patienten mit entferntem Kehlkopf eine Prothese verwendet werden, bei welcher Luft aus der Lunge über ihren natürlichen Strömungsweg durch ein Tracheostoma nach Außen gelangt, wobei die Stimmprothese (Tracheostoma- Tubus mit Sprechventil) einen mehr oder weniger dauerhaften Durchlass in die Speiseröhre schafft. Dadurch ergibt sich das s.g. oesophagale Sprechen.

Die Tracheostoma- Tuben mit Sprechventil bestehen zumeist aus einer Trachealkanüle mit Kanülenschild und einem Ventilaufnahmeteil zur Halterung eines äußeren Sprechventils, wobei ein inneres Sprechventil der Trachealkanüle in einer Röhre zwischen Speiseröhre und Luftröhre gelagert ist, so dass bei blockiertem Tracheostoma (bei verschlossenem äußeren Sprechventil) Luft durch das innere Sprechventil in Richtung Mundraum entweichen kann und dabei im oberen Bereich der Speiseröhre Schwingungen zum Sprechen erzeugt werden. Das Blockieren des Tracheostoma erfolgt dabei manuell durch Verschließen des äußeren Sprechventils an der Außenseite der Trachealkanüle.

Diese Tracheostoma- Prothesen umfassen meist flexible Haltekrägen. Der Haltekragen liegt bei der bestimmungsgemäßen Verwendung der Stimmprothese an der Speiseröhren- seitigen Oberfläche der Scheidewand zwischen Luft- und Speiseröhre an, um die Wahrscheinlichkeit eines Verlagerns der Prothese im Tracheostoma möglichst zu vermeiden oder zu vermindern.
Dieser Haltekragen verbessert somit das Halten der Prothese wesentlich (schützt bspw. vor dem Aushusten der Prothese), hat jedoch den Nachteil, dass ein großer Haltekragen das Einsetzen einer Prothese erschwert, in dem das Tracheostoma- umgebende Gewebe traumatisiert wird.

Aus EP 1 099 451 A3 ist die Verwendung von konischen Spitzen bei der Tracheostomie grundsätzlich bekannt.

Aus DE 10 2005 021 470 A1 ist eine Einführhilfe für die perkutane Tracheostomie bekannt, die einen durch die Trachealkanüle hindurchführbaren Schaft mit konischer Spitze aufweist, die in einem ersten Zustand einen kleines Basisdurchmesser und in einem zweiten Zustand einen großen Basisdurchmesser besitzt, wobei die Einführhilfe einen an die konische Spitze angernzenden Abschnitt hat, dessen Durchmesser maximal dem Innendurchmesser einer mit der Einführhilfe einzuführenden Trachealkanüle entspricht.

DE 694 32 755 T2 offenbart eine Kunststoffeinführhilfe für die Tracheostomie, bei welcher zwischen einzelnen Vorsprüngen flexible Biegungsabschnitte für die bessere Handhabbarkeit vorgesehen sind.

Aus WO87/06817 ist eine Prothese zur Sinusdrainage bekannt, die flexible Flügel aufweist, welche gefaltet werden können, um die Prothese entlang eines Körperkanals zu bewegen.

DE 20 2010 002 803 U1 offenbart eine Vorrichtung zum Einbringen einer Trachealkanüle in ein Tracheostoma mit einer Einführhilfe (Inserter), wobei die Spitze der Einführhilfe mit einem Gleitschirm stoffschlüssig verbunden ist, und sicher der Gleitschirm beim Hineinschieben der Einführhilfe in das Trachealkanülenrohr mit einer Abdeckbreite über die Kanülenrohrkante legt.

DE 693 17 445 T2 offenbart eine Vorrichtung zum Einsetzen einer Prothese in ein Loch zwischen Luft- und Speiseröhre (Tracheostoma) zur Wiederherstellung der Stimme, welche aus einem zylindrischen Vorrichtungskörper mit einer Längsachse, einem auf einer äußeren Oberfläche des Vorrichtungskörpers vorgesehenen flexiblen Flansch, der eine Gebrauchsrichtung hat, in der er sich im wesentlichen von der äußeren Oberfläche des Vorrichtungskörpers nach außen erstreckt und eine Einsatzrichtung hat, in der er nachgiebig auf die Längsachse des Vorrichtungskörpers zu gefaltet ist, und eine Einführhilfe mit einem Mittel (bspw. Gelatinekapsel) zum Halten des Flansches in seiner nachgiebig gefalteten Ausrichtung besteht, wobei die Haltemittel aus einem Material (bspw. Gelatine) bestehen, das in der Flüssigkeit, welche sich in der Scheidewand zwischen Speise- und Luftröhre befindet, löslich ist.

Der Nachteil dieser Erfindung ist, dass die flüssigkeitslöslichen Stoffe (Gelatinekapseln) als körperfremde Stoffe Reizungen oder Immunreaktionen bei dem Patienten hervorrufen oder dass sich die Kapseln (bspw. Gelatinekapseln) nicht vollständig auflösen, so dass der Patient beim oesophagalen Sprechen sehr viel Kraft zum Erreichen eines Luftstromes durch die Prothese aufwenden muss.

Der Erfindung liegt die Aufgabe zugrunde, eine Vorrichtung zum Einsetzen in ein Loch zwischen Luft- und Speiseröhre anzugeben, welche die Nachteile des Standes der Technik vermeidet, insbesondere eine richtige Positionierung der Vorrichtung im Patienten in Gebrauchsausrichtung mit optimalen Luftstrom ermöglicht, ohne dass Reizungen bzw. Immunreaktionen bei dem Patienten auftreten und ohne dass die Gefahr des Aushustens durch den Patienten besteht.

Gelöst wird diese Aufgabe durch eine Vorrichtung zum Einsetzen in ein Tracheostoma gemäß dem ersten Patentanspruch. Vorteilhafte Ausgestaltungen der Erfindung sind in den nachgeordneten Ansprüchen angegeben.

Das Wesen der Erfindung besteht in der Bereitstellung einer neuartigen Vorrichtung zum Einsetzen in ein Tracheostoma, welche durch flexible Flügel, die durch eine Einführhilfe am proximalen Ende der Vorrichtung im gefalteten Zustand gehaltert werden, optimal in ein Tracheostoma eingeführt und dort durch Entfalten der Flügel positioniert werden kann.

Der Vorteil der erfindungsgemäßen Vorrichtung zum Einsetzen in ein Tracheostoma besteht darin, dass durch sie eine richtige Positionierung der Vorrichtung im Patienten in Gebrauchsausrichtung mit optimalen Luftstrom ermöglicht ist, ohne dass Reizungen bzw. Immunreaktionen bei dem Patienten auftreten und ohne dass die Gefahr des Aushustens durch den Patienten besteht.

Die Erfindung wird nachstehend an Hand des Ausführungsbeispiels und der Figur näher erläutert. Dabei zeigt:

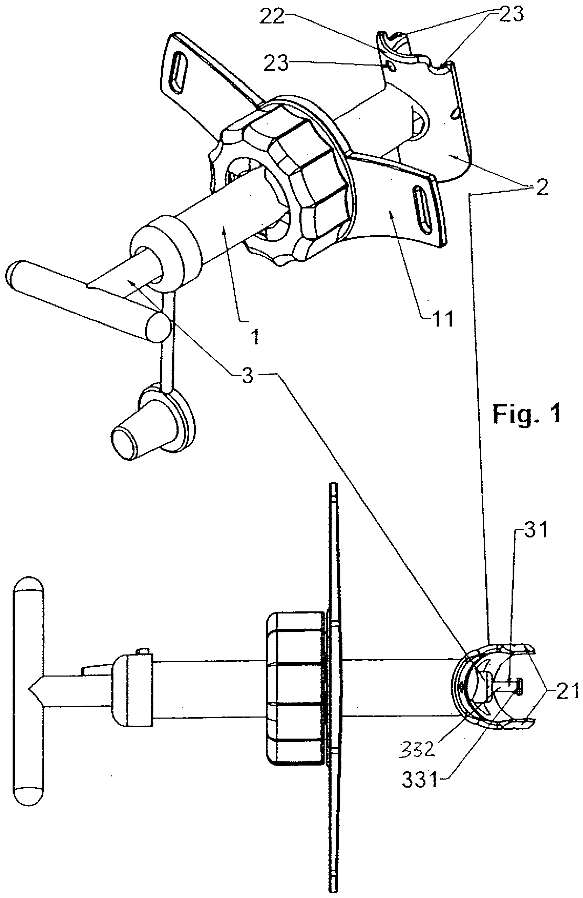

- Fig. 1:: eine schematische 3-D-Darstellung einer Ausführungsform einer erfindungsgemäßen Vorrichtung.

Fig. 1 zeigt eine Vorrichtung zum Einsetzen in ein Tracheostoma umfassend einen zylindrischen Vorrichtungskörper (1) mit einer Längsachse und einem auf einer äußeren Oberfläche des Vorrichtungskörpers (1) vorgesehenen flexiblen Flansch (2), der eine Gebrauchsrichtung hat, in der er sich im wesentlichen von der äußeren Oberfläche des Vorrichtungskörpers (1) nach außen erstreckt und eine Einsatzrichtung hat, in der er nachgiebig auf die Längsachse des Vorrichtungskörpers (1) zu gefaltet ist, und eine stiftförmige Einführhilfe (3) mit einem Mittel (31) zum Halten des Flansches (2) in seiner nachgiebig gefalteten Ausrichtung.
Gemäß der Erfindung ist der Flansch (2) aus zumindest zwei Flügeln (21) gebildet, welche im Bereich der Flügelspitze (22) mit einer Ausnehmung (23) versehen sind, in welche das Mittel (31) der Einführhilfe (3) lösbar eingeführt ist. Dabei bestehen zwischen den Flügeln (21) des Flansches (2) Luftzirkulations- Freiräume (221).

Die so gefalteten Flügel (21) ein Formgedächtnis auf, welches die Flügel (21) von der Längsachse des Vorrichtungskörpers (1) weg orientiert.

Die Ausnehmungen (23) der Flügel (21) sind rund und das Mittel (31) der Einführhilfe (3) besitzt eine kugelförmige Spitze (331), die über eine Verbindung (332) mit geringem Durchmesser das proximale Ende der Einführhilfe (3) bildet, wobei der Außendurchmesser der Einführhilfe (3) und der Spitze (331) maximal dem Innendurchmesser des Vorrichtungskörpers (1) entspricht.
Der Rand der Ausnehmungen (23) ist geschlitzt, so dass ein Ein- und Ausführen der Mittel (31) erleichtert ist.

Der Vorrichtungskörper (1) besteht aus nachgiebigem Silikon, wobei die Flügel (21) ausreichend flexibel sind, um faltbar zu sein.

Der Vorrichtungskörper (1) weist auf seiner distalen Seite einen Stopper (11) in Form eines Schildes auf, welcher auf dem Vorrichtungskörper (1) entlang der Längsachse in proximale Richtung verschieb- und arretierbar ist, wobei das Arretieren durch eine Mutter am Stopper (Schraubschild) erfolgt, in dem der Innendurchmesser des Stoppers auf dem Vorrichtungskörper (1) durch Verdrehen verengt wird. Dadurch ist die Einsetztiefe in Einsetzrichtung individuell einstellbar ist.

Bei Bedienen der Vorrichtung wird die stiftförmige Einführhilfe (3) in den zylindrischen Vorrichtungskörper (1) eingeführt wird, wobei die Flügel (21) des Flansches (2) in Einsetzrichtung entlang der Längsachse des Vorrichtungskörpers (1) gefaltet und dabei die Ausnehmung (23) im Bereich der Flügelspitzen (22) über die kugelförmige Spitze (331) des Mittels (31) zum Halten des Flansches (2) geführt werden.
Zum Auffalten der Flügel (21) des Vorrichtungskörpers (1) in Gebrauchsausrichtung wird die Einführhilfe (3) aus dem Vorrichtungskörper (1) herausgezogen wird, so dass die Flügel (21) des Flansches (2) entfaltet werden.

Die erfindungsgemäße Vorrichtung ist in einem Tracheostoma eines Patienten einführbar.

### Bezugszeichenliste

- 1: - Vorrichtungskörper
- 2: - Flansch
- 21: - Flügel
- 22: - Flügelspitze
- 221: - Luftzirkulationsfreiraum
- 23: - Ausnehmung
- 3: - Einführhilfe
- 31: - Mittel zum Halten
- 331: - kugelförmige Spitze
- 332: - Verbindung

## Patentansprüche

1. Vorrichtung zum Einsetzen in ein Tracheostoma umfassend einen zylindrischen Vorrichtungskörper (1) mit einer Längsachse und einem auf einer äußeren Oberfläche des Vorrichtungskörpers (1) vorgesehenen flexiblen Flansch (2), der eine Gebrauchsrichtung hat, in der er sich im wesentlichen von der äußeren Oberfläche des Vorrichtungskörpers (1) nach außen erstreckt und eine Einsatzrichtung hat, in der er nachgiebig auf die Längsachse des Vorrichtungskörpers (1) zu gefaltet ist, und eine stiftförmige Einführhilfe (3) mit einem Mittel (31) zum Halten des Flansches (2) in seiner nachgiebig gefalteten Ausrichtung, **dadurch gekennzeichnet, dass** der Flansch (2) aus zumindest zwei Flügeln (21) gebildet ist, welche im Bereich der Flügelspitze (22) mit einer Ausnehmung (23) versehen sind, in welche das Mittel (31) lösbar eingeführt ist, und dass die Flügel (21) ein Formgedächtnis aufweisen, welches die Flügel (21) von der Längsachse des Vorrichtungskörpers (1) weg orientiert.

2. Vorrichtung zum Einsetzen in ein Tracheostoma gemäß Anspruch 1, **dadurch gekennzeichnet, dass** die Ausnehmungen (23) rund sind und dass das Mittel (31) eine kugelförmige Spitze (331) besitzt, die über eine Verbindung (332) mit geringen Durchmesser das proximale Ende der Einführhilfe (3) ausbildet, wobei der Außendurchmesser der Einführhilfe (3) und der Spitze (331) maximal dem Innendurchmesser des Vorrichtungskörpers (1) entspricht.

3. Vorrichtung zum Einsetzen in ein Tracheostoma gemäß Anspruch 1 oder 2, **dadurch gekennzeichnet, dass** der Vorrichtungskörper (1) aus nachgiebigen Silikon besteht.

4. Vorrichtung zum Einsetzen in ein Tracheostoma gemäß Anspruch 3, **dadurch gekennzeichnet, dass** Flügel (21) ein Formgedächtnis aufweisen und ausreichend flexibel sind, um faltbar zu sein.

5. Vorrichtung zum Einsetzen in ein Tracheostoma gemäß Anspruch 2, 3 oder 4, **dadurch gekennzeichnet, dass** der Rand der Ausnehmungen (23) geschlitzt ist.

6. Vorrichtung zum Einsetzen in ein Tracheostoma gemäß Anspruch 1, **dadurch gekennzeichnet, dass** der Vorrichtungskörper (1) auf seiner distalen Seite einer Stopper (11) in Form eines Schildes aufweist, welcher auf dem Vorrichtungskörper (1) entlang der Längsachse in proximale Richtung verschieb- und arretierbar ist.

7. Vorrichtung zum Einsetzen in ein Tracheostoma gemäß Anspruch 1, **dadurch gekennzeichnet, dass** zwischen den Flügeln (21) des Flansches (2) Luftzirkulations- Freiräume (22) bestehen.

## Claims

1. A device for insertion in a tracheostoma comprising a cylindrical device body (1) with a longitudinal axis and a flexible flange (2), which is provided on an outer surface of the device body (1) and has a direction of insertion in which it extends mainly from the outer surface of the device body (1) to the outside and a direction of use in which it is flexibly folded towards the longitudinal axis of the device body (1), and a pin-shaped insertion aid (3) having a means (31) for holding the flange (2) in its flexibly folded orientation, **characterized in that** the flange (2) is formed by at least two wings (21) having a recess (23) in the region of the wing tip (22), in which the means (31) is removably introduced, and that the wings (21) have a shape memory that orientates the wings (21) away from the longitudinal axis of the device body (1).

2. The device for insertion in a tracheostoma according to claim 1, **characterized in that** the recesses (23) have a round shape and that the means (31) is provided with a spherical tip (331) forming the proximal end of the insertion aid (3) via a connection (332) with a small diameter, wherein the maximum possible value of the external diameters of the insertion aid (3) and of the tip (331) corresponds to the value of the internal diameter of the device body (1).

3. The device for insertion in a tracheostoma according to claim 1, **characterized in that** the device body (1) is made of flexible silicone.

4. The device for insertion in a tracheostoma according to claim 3, **characterized in that** wings (21) have a shape memory and are flexible enough to be folded.

5. The device for insertion in a tracheostoma according to claim 2, 3 or 4, **characterized in that** the edge of the recesses (23) is slotted.

6. The device for insertion in a tracheostoma according to claim 1, **characterized in that** the device body (1) on its distal side is provided with a stopper (11) in form of a shield which can be shifted on the device body (1) along the longitudinal axis in proximal direction and locked in place.

7. The device for insertion in a tracheostoma according to claim 1, **characterized in that** air circulation spaces (22) are provided between the wings (21) of the flange (2).

## Revendications

1. Dispositif destiné à être placé dans un trachéostome contenant un corps de dispositif cylindrique (1) avec un axe longitudinal et une bride flexible (2) prévue sur la surface extérieure du corps de dispositif (1), ladite bride ayant une direction d'utilisation, dans laquelle elle s'étend essentiellement de la surface extérieure du corps de dispositif (1) vers l'extérieur, et une direction d'insertion, dans laquelle elle est souplement pliée vers l'axe longitudinal du corps de dispositif (1), et un aide à insertion (3) en forme de pointe avec un instrument (31) pour maintenir la bride (2) dans son extension souplement pliée, est **caractérisé en ce que** la bride (2) se compose au moins de deux ailes (21) prévues d'un évidement (23) dans la zone de la pointe d'aile (22), dans laquelle l'instrument (31) est inséré de façon amovible, et que les ailes (21) possèdent une mémoire de forme, qui oriente les ailes (21) vers le côté opposé de l'axe longitudinal du corps de dispositif (1).

2. Dispositif destiné à être placé dans un trachéostome suivant la revendication 1 est **caractérisé en ce que** les évidements (23) sont ronds et que l'instrument (31) possèdent une pointe sphérique (331) formant l'extrémité proximale de l'aide à introduction (3) par une connexion (332) à faible diamètre et le diamètre extérieur de l'aide à introduction (3) et de la pointe (331) correspondant au maximum au diamètre intérieur du corps de dispositif (1).

3. Dispositif destiné à être placé dans un trachéostome suivant les revendications 1 ou 2 est **caractérisé en ce que** le corps de dispositif (1) se compose de silicone souple.

4. Dispositif destiné à être placé dans un trachéostome suivant la revendication 3 est **caractérisé en ce que** les ailes (21) possèdent une mémoire de forme et flexibilité suffisante pour être pliable.

5. Dispositif destiné à être placé dans un trachéostome suivant les revendications 2, 3 ou 4 est **caractérisé en ce que** le bord des évidements (23) est fendu.

6. Dispositif destiné à être placé dans un trachéostome suivant la revendication 1 est **caractérisé en ce que** le corps de dispositif (1) est muni d'une butée (11) sous forme d'un bouclier sur son côté distal, qui peut être déplacé et bloqué sur le corps de dispositif (1) le long de l'axe longitudinal en direction proximale.

7. Dispositif destiné à être placé dans un trachéostome suivant la revendication 1 est **caractérisé en ce que** des espaces libres pour la circulation d'air (22) existent entre les ailes (21) de la bride (2).
